# EUROPÄISCHE PATENTSCHRIFT

(11) **EP 2 316 390 B1**
(45) Veröffentlichungstag und Bekanntmachung des Hinweises auf die Patenterteilung: **14.03.2012**
(21) Anmeldenummer: 10188705.7
(22) Anmeldetag: 25.10.2010
(51) Int. Cl.: A61F 2/64, A61F 2/68

(54) **Kniegelenk für eine Prothese**
Knee joint for a prosthetic
Genouillère pour une prothèse

(30) Priorität: 02.11.2009 DE 102009051668
(43) Veröffentlichungstag der Anmeldung: 04.05.2011
(73) Patentinhaber: medi GmbH & Co. KG, 95448 Bayreuth (DE); PRO LIMB International Corp., Shulin City 23877 (TW)
(72) Erfinder: Hsin, Fa Shen, 220, Banquao City (TW)
(74) Vertreter: Blaumeier, Jörg

(56) Entgegenhaltungen:
- EP-A1- 0 700 491
- EP-A1- 1 570 817
- WO-A2-2007/025116
- DE-U1- 20 306 821
- US-A1- 2002 026 246
- KAPHINGST UND G STARK W: "Polyzentrische Gelenke in der Prothetik - Eigenschaften und Einsatzbereiche", ORTHOPAEDIE-TECHNIK, BUNDESINNUNGSVERBAND FUER ORTHOPAEDIE-TECHNIK, DE, Nr. 7, 1. Januar 2001 (2001-01-01), Seiten 484-490, XP009143911, ISSN: 0340-5591

## Beschreibung

Die Erfindung betrifft ein Kniegelenk für eine Prothese, umfassend eine Schwungphasensteuerung mit einem mit einem Oberschenkelverbindungsstück gekoppelten, gegen einen Zylinder bewegbaren Kolben und dem mit einem Oberschenkelverbindungsstück gekoppelten Zylinder, wobei der Zylinder gegen das Unterschenkelverbindungsstück um wenigstens eine Achse verschwenkbar ist.

Gelenke mit einer sogenannten Schwungphasensteuerung sind im Stand der Technik bereits bekannt. Eine Schwungphasensteuerung dient dem Zweck, die im in einer Prothese vorgesehenen Kniegelenk auftretenden Kräfte beim Gehen so zu nutzen, dass eine möglichst dem mit einem natürlichen Gelenk durchgeführten Gehvorgang entsprechende Beugung (Flexion) des Knies auftritt (sogenannte Schwungphase). Dazu umfasst die Schwungphasensteuerung üblicherweise einen in einem Zylinder geführten Kolben, der gegenüber dem Zylinder bewegbar ist. Der Bewegungsfreiraum des Kolbens innerhalb des Zylinders definiert dabei beispielsweise zwei Kammern, wobei es bekannt ist, beispielsweise über die Ausgestaltung der Luftwege oder das Vorsehen zusätzlicher Mittel, beispielsweise Federn, die Schwungphase möglichst einer tatsächlichen Gehbewegung und dem Komfortbedürfnis des die Prothese Nutzenden entgegenkommend zu steuern.

Ein wichtiges Element in heutigen Kniegelenken ist die Sicherung der Standphase, das bedeutet, das möglichst weitgehende Vermeiden einer Beugung des Kniegelenks, wenn das Kniegelenk in der Standphase belastet ist. Dafür ist es notwendig, eine das Kniegelenk in die Extensionsstellung zurückdrängende Kraft zu erzeugen. Dazu wurde beispielsweise in der EP -0 700 491 B1 vorgeschlagen, in einer durch den Kolben im Zylinder definierten, unterschenkelseitigen zweiten Kammer eine sich an einem Fußteil des Zylinders abstützende, den Kolben zum Kopfteil des Zylinders hin vorspannende Feder vorzusehen. Im Betrieb wird dabei die Flexionsbewegung durch die Feder gedämpft, die Extensionsbewegung hingegen wird durch die Feder beschleunigt.

Dies hat jedoch, insbesondere auch bei polyzentrischen Kniegelenke, den Nachteil, dass mit zunehmender Flexion eine zunehmende Vorspannung der Feder erfolgt, so dass eine ständig ansteigende Kraft, die in Richtung der Extension wirkt, entsteht. Dies beeinträchtigt jedoch die Funktion der Schwungphasensteuerung, da permanent ansteigende, in die Extensionsrichtung wirkende Kräfte erzeugt werden. Zudem wird die Funktion der Standphasensicherung mit einer deutlich ansteigenden Kraft letztlich nur bei kleinen Kniegelenksbeugewinkeln (Flexionswinkeln, also der Winkel, den das Oberschenkelteil und das Unterschenkelteil zueinander einnehmen) benötigt.

Gerade bei polyzentrischen Kniegelenken für Prothesen ist ein Kraftverlauf wichtig und wünschenswert, der bei kleinen Winkeln stark ansteigt, da nur ein maximal gestrecktes polyzentrisches Prothesenkniegelenk bei Fußkantakt nicht einbeugt.

Polyzentrische Gelenke sind im Stand der Technik ebenso grundsätzlich bekannt, vgl. hierzu beispielsweise den Artikel "Polyzentrische Gelenke in der Prothetik - Eigenschaften und Einsatzbereiche" von W. Kaphingst und G. Stark, in: Orthopädie-Technik 7/01, S. 484 - 490. Sie zeichnen sich dadurch aus, dass die Flexionsbewegung letztlich nicht durch eine, ein Oberschenkelverbindungsstück mit einem Unterschenkelverbindurigsstück verbindende Achse erfolgt, sondern dass die Bewegung über mehrere Achsen vermittelt wird, so dass ein komplexerer, eher dem gewünschten Verhalten des Kniegelenks angepasster Bewegungsablauf erfolgen kann. Üblicherweise sind dabei das Oberschenkelverbindungsstück und das Unterschenkelverbindungsstück über wenigstens ein Zwischenstück gekoppelt, wobei Verbindungen über Achslagerungen hergestellt werden können. Eine bekannte Form eines polyzentrischen Gelenks ist beispielsweise die sogenannte Vierachsgeometrie. Dabei werden das Oberschenkelverbindungsstück (Kniekopf) und das Unterschenkelverbindungsstück (Sitz zur Verbindung mit einem Unterschenkelteil) über ein erstes, kürzeres und ein zweites, längeres Zwischenstück aneinandergekoppelt sind. Ein derartiges Vierachsgelenk ist beispielsweise aus der WO 03/092545 A2 bekannt.

Aus US 2002/0026246 A1 ist ein Kniegelenk mit fünf gelenkig miteinander verbundenen Verbindungsabschnitten bekannt, wobei zwischen zwei im unteren Kniegelenkbereich vorgesehenen, miteinander verbundenen Gelenkabschnitten ein elastisches Element vorgesehen ist, mittels dem der über diese beiden Verbindungsabschnitte gebildete Winkel vergrößert wird.

Aus EP 1 570 817 A1 ist ein Kniegelenk mit Bremsfunktion bekannt, bei dem es möglich ist, mechanisch zu erfassen, welcher Teil des Fußes belastet wird. Hierzu ist ein Verbindungsmechanismus vorgesehen, mittels dem die Lasterfassung erfolgt. Abhängig von der Lasterfassung wird sodann eine hydraulische Bremse gesteuert.

WO 2007/025116 beschreibt eine Beinprothese mit einem aktiven Kniegelenk mit zugeordnetem Aktuator und einer Dämpfungseinrichtung, die über ein elektronisches Steuersystem gesteuert werden.

Schließlich ist aus DE 203 06 821 U1 eine künstliche Kniegelenksanordnung bekannt, welche in der Lage ist, einen Kniewinkel zwischen einem Unterschenkel und einem Oberschenkel aufrecht zu erhalten, wenn die Kniegelenkanordnung auf einer horizontalen oder geneigten Oberfläche steht.

Dabei kann der Kolben der Schwungphasensteuerung über seine Kolbenstange unmittelbar schwenkgelagert mit dem Oberschenkelverbindungsstück verbunden sein, während der Zylinder schwenkgelagert am längeren, zweiten Zwischenstück angeordnet ist. Es ist wichtig, hervorzuheben, dass die Schwenkachsen, die die Schwungphasensteuerung an die Bestandteile des Kniegelenks koppeln, im Allgemeinen nicht den Achsen entsprechen, mit dem die einzelnen Teilstücke des Kniegelenks im Rahmen der polyzentrischen Geometrie, beispielsweise einer Vierachsgeometrie, miteinander verbunden sind.

Die Begriffe Oberschenkelverbindungsstück, Unterschenkelverbindungsstück und Zwischenstück sind dabei so zu verstehen, dass sie durchaus aus mehreren Bestandteilen bestehen können, welche jedoch starr miteinander verbunden oder starr bewegungsgekoppelt sind und somit im Kniegelenk eine starre Bewegungseinheit bilden. Beispielsweise sind auch Zwischenstücke bekannt, die aus zwei gegenüberliegend die entsprechenden Achslagerungen begrenzenden Verbindungsplatten bestehen.

Der Erfindung liegt daher die Aufgabe zugrunde, ein polyzentrisches Kniegelenk für eine Prothese anzugeben, in dem eine Sicherung der Standphase mit einem besser diesem Zweck angepassten Kraftverlauf möglich ist.

Zur Lösung dieser Aufgabe ist bei einem Kniegelenk der eingangs genannten Art erfindungsgemäß vorgesehen, dass der Zylinder über einen achsfernen Anlenkpunkt an ein sich an dem Unterschenkelverbindungsstück abstützendes Federmittel gekoppelt ist.

Die Erfindung schlägt also vor, ein Federmittel nicht mehr, wie bekannt, innerhalb der Schwungphasensteuerung vorzugehen, um die Standphase zu sichern, sondern schlägt ein sich am Unterschenkelverbindungsstück abstützendes Federmittel vor, welches die Verschwenkung des Zylinders gegenüber dem Unterschenkelverbindungsstück, mit welcher folglich eine Änderung des Abstands des achsfernen Anlenkpunktes einhergeht, ausnutzt, um hierüber ein der Verschwenkung der beginnenden Flexionsbewegung entgegenwirkendes Drehmoment aufzubauen, welches mithin eine in Extensionsrichtung wirkende Kraft erzeugt, die zur Sicherung der Standphase führt.

Die Erfindung richtet sich mithin hauptsächlich auf polyzentrische Kniegelenke, bei denen der Bewegungsablauf zu Beginn der Flexionsbewegung ein relativ starkes Verschwenken des Zylinders gegenüber dem Unterschenkelverbindungsstück bedingt, wobei während des folgenden Bewegungsablaufs nur noch geringe Verschwenkungen in dieser Richtung auftreten oder gar die Verschwenkung wieder aufgehoben wird. Denn die Sicherung der Standphase ist vor allem in niedrigen Winkelbereichen wünschenswert, während in höheren Winkelbereichen zum möglichst störungsfreien Betrieb der Schwungphasensteuerung eine weiter stark ansteigende Gegenkraft, wie sie durch eine Feder in der Schwungphasensteuerung selbst erzeugt würde, nicht mehr erwünscht ist. Ein solcher Bewegungsablauf kann bei verschiedenen polyzentrischen Kniegelenken auftreten, worauf im Folgenden noch näher eingegangen werden wird.

Die vorliegende Erfindung nutzt folglich eine Abstandsänderung des Anlenkpunktes zu einem Abstützpunkt auf dem Unterschenkelverbindungsstück, um die die Standphase sichernde Kraft zu erzeugen. So wird die maximale Extension unterstützt, so dass ein polyzentrisches Kniegelenk für eine Prothese, bei welchem die Standphase derart gesichert ist, bei Fußkontakt nicht einbeugt.

Es sei an dieser Stelle darauf hingewiesen, dass die schwenkbare Ankopplung des Zylinders an das Unterschenkelverbindungsstück nicht unmittelbar über eine einzige Achse, mithin eine einzige Achslagerung, erfolgen muss, sondern dass auch polyzentrische Kniegelenke, insbesondere in der eingangs genannten Vierachsgeometrie, bekannt sind, bei denen zunächst das Unterschenkelverbindungsstück über eine erste, einer ersten Schwenkachse zugeordnete Achslagerung mit einem Zwischenstück verbunden ist, wobei der Zylinder über eine zweite, einer zweiten Schwenkachse entsprechende Achslagerung, welche insbesondere benachbart zu der ersten Achslagerung und entfernt zu einer dritten Achslagerung, mit der das Zwischenstück mit dem Oberschenkelverbindungsstück oder einem weiteren Zwischenstück verbunden ist, angeordnet ist, verbunden ist. Je nachdem, welche Achse - also die erste und/oder die zweite Achse - für die Schwenkbewegung bei der beginnenden Flexionsbewegung verantwortlich ist, ist der Anlenkpunkt idealerweise so angeordnet, dass das Federmittel bei der betreffenden Bewegung am effektivsten ein Drehmoment erzeugen kann.

Wenn von einer Mehrachsgeometrie gesprochen wird, beispielsweise von einer Vierachsgeometrie, so werden im Allgemeinen die die verschiedenen Teilstücke des Kniegelenks verbindenden Achsen - im oberen Beispiel also die erste und die dritte Achse - "gezählt". Sie sind es häufig auch, die zu Beginn einer Flexionsbewegung zuerst beansprucht werden.

Es kann mithin allgemein vorteilhafterweise vorgesehen sein, dass bei einer Flexionsbewegung in einem vorgegebenen ersten, bei 0° beginnenden Flexionswinkelintervall, insbesondere einem bei einem Winkel kleiner als 20°, bevorzugt kleiner als 10°, endenden Flexionswinkelintervall, eine Verschwenkung des Zylinders um die oder wenigstens eine Achse erfolgt und so eine ein gegenläufiges Drehmoment erzeugende Vorspannkraft in dem Federmittel aufbaubar ist. Ein besonders vorteilhafter Kraftverlauf ergibt sich, wenn in einem an das erste Flexionsintervall anschließenden zweiten, insbesondere bei 90° endenden flexionswinkelintervall keine wesentliche Verschwenkung des Zylinders mehr auftritt. Auf diese Weise ist, wie oben beschrieben, ein Kraftverlauf realisiert, bei dem im relevanten Bereich, also bei niedrigen Winkeln, schnell eine hinreichend starke Vorspannungskraft aufgebaut wird, die die Standphase sichert. Nach dem ersten, kleinen Flexionswinkelintervall bleibt die das gegenläufige Drehmoment erzeugende Vorspannkraft jedoch im Wesentlichen konstant, da nur noch Verschwenkungen auftreten, die, wenn überhaupt, nur eine sehr viel geringere Änderung des Abstands zwischen dem Anlenkpunkt und dem Abstützbereich auf dem Unterschenkelverbindungsstück bedingt. So wird eine Beeinträchtigung der Schwungphasensteuerung durch sich permanent ändernde, insbesondere permanent ansteigende, in die Extensionsrichtung drückende Kräfte vermieden.

Dabei einer Verschwenkung bzw. sonstigen Bewegung, beispielsweise Verschiebung, des Zylinders nicht nur eine Abstandsänderung, sondern auch eine Verdrehung auftritt, ist es von besonderem Vorteil, wenn an dem Federmittel eine Gelenkverbindung zum Ausgleich einer relativen Bewegung zwischen dem Unterschenkelverbindungsstück und dem Zylinder vorgesehen ist. So beeinflussen diese relativen Verschwenkungen die grundsätzliche Funktion des Federmittels nicht. In konkreter Ausgestaltung kann beispielsweise vorgesehen sein, dass die Gelenkverbindung eine an dem Unterschenkelverbindungsstück angeordnete, eine insbesondere kalottenförmige Mulde umfassende Gelenkpfanne und einen insbesondere kalottenförmigen, in die Mulde einzusetzenden Gelenkkörper umfasst. Auf diese Weise wird ein Kugelgelenk geschaffen, das diese Verschwenkungen ausgleichen kann.

Besonders vorteilhaft können die Gelenkpfanne und der Gelenkkörper eine Durchgangsöffnung für eine an dem Anlenkpunkt mit dem Zylinder gelenkig verbundene Stange aufweisen, um welche Stange sich das als wenigstens eine Feder, insbesondere eine Schraubenfeder, ausgebildete, sich auf einer der Mulde abgewandten Fläche des Gelenkkörpers und einem an dem dem Zylinder abgewandten Ende der Stange vorgesehenen Anschlagmittel abstützende Federmittel erstreckt. Auf diese Weise ist es möglich, das Federmittel an einer dem Zylinder grundsätzlich abgewandten Oberfläche des Unterschenkelverbindungsstücks anzuordnen, die dann entsprechend auch eine Durchgangsöffnung für die Stange aufweist, beispielsweise im Inneren einer Rohraufnahme für den Unterschenkelteil bzw. Fußteil der Prothese, so dass das Federmittel und/oder die Gelenkverbindung und/oder das Anschlagmittel mit besonderem Vorteil verdeckt und geschützt angeordnet sein können. So kann beispielsweise eine dem Anlenkpunkt benachbarte Fläche des Unterschenkelverbindungsstücks, die Teil der Wandung einer Rohraufnahme darstellt, eine Durchgangsöffnung aufweisen. Innerhalb der Rohraufnahme stützt sich dann von der anderen Seite gegen diese Fläche die Gelenkverbindung mit der Gelenkpfanne und dem in der Mulde angeordneten Gelenkkörper ab. Durch die Durchgangsöffnungen der Fläche, der Gelenkpfanne und des Gelenkkörpers ragt die am Anlenkpunkt gelenkig verbundene Stange hindurch, wobei die Durchgangsöffnung des Gelenkkörpers insbesondere so ausgebildet ist, dass sie die Stange formschlüssig umschließt. Auf der anderen Seite ragt die Stange über den Gelenkkörper hinaus und weist schließlich einen Anschlag auf. Auf dem Anschlag und einer dem Anschlag zugewandten, insbesondere ebenen Oberfläche des Gelenkkörpers stützt sich nun das als eine oder mehrere Federn ausgebildete Federmittel ab. Neben einer Schraubenfeder ist es auch denkbar, beispielsweise mehrere hintereinander angeordnete Blattfedern zu verwenden. Wird nun der Abstand zwischen dem Anlenkpunkt und dem Unterschenkelverbindungsstück größer, so wird die Feder zusammengepresst, so dass eine Vorspannkraft erzeugt werden kann. Selbstverständlich ist auch der umgekehrte Fall denkbar, dass zu Beginn einer Flexionsbewegung eine Verringerung des Abstands auftritt, so dass die Feder gedehnt wird und auf diese Weise eine entgegenwirkende Vorspannkraft entsteht.

In vorteilhafter weiterer Ausgestaltung kann bei Verwendung einer Stange die Stange als Gewindestange und das Anschlagmittel als auf dem Gewinde verstellbare Schraubmutter ausgebildet sein. So ist es möglich, die grundsätzlich vorhandene Vorspannung und somit auch die später auftretenden Kräfte ideal einzustellen.

Doch es ist im Rahmen der vorliegenden Erfindung auch allgemein denkbar, dass die Vorspannung des Federmittels einstellbar ist. So können die bei einer flexionsbewegung entstehenden, in Richtung der Extension wirkenden Kräfte bzw. Drehmomente ideal den gewünschten Voraussetzungen angepasst werden, sowohl für die spezielle Art des polyzentrischen Kniegelenks als auch patientenindividuell.

Weitere Vorteile, Merkmale und Einzelheiten der vorliegenden Erfindung ergeben sich aus dem im Folgenden beschriebenen Ausführungsbeispiel sowie anhand der Zeichnung. Dabei zeigen:
- Fig. 1: ein erfindungsgemäßes Kniegelenk in der maximalen Extensionestel- lung,
- Fig. 2: das erfindungsgemäße Kniegelenk in einer leicht gebeugten Stel- lung, und
- Fig. 3: das erfindungsgemäße Kniegelenk in einer stark gebeugten Stellung.

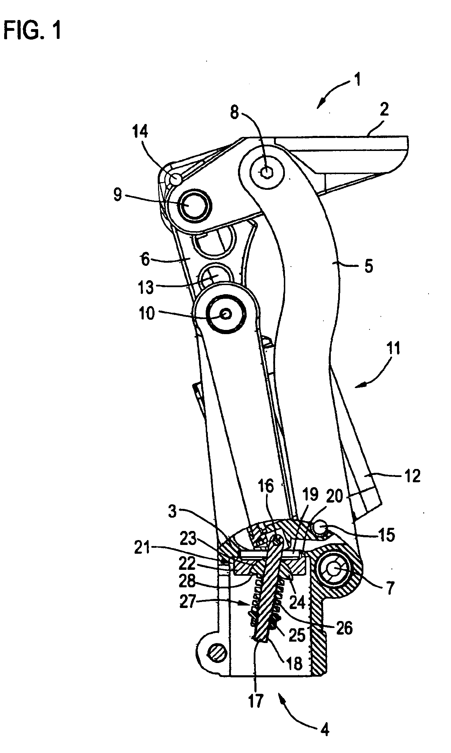

Fig. 1 zeigt ein erfindungsgemäßes polyzentrisches Kniegelenk 1. Es umfasst ein Oberschenkelverbindungsstück 2 (Knieknopf) und ein Unterschenkelverbindungsstück 3, wobei ein Prothesenschaft des Oberschenkels an dem Oberschenkelverbindungsstück 2 angeschlossen werden kann, und ein Unterschenkelteil bzw. Fußteil der Prothese an dem Unterschenkelverbindungsstück 3, wofür dieses insbesondere eine Rohraufnahme 4 umfasst. Es sind jedoch selbstverständlich auch andere Möglichkeiten denkbar, um das Fußteil einer Prothese mit dem Kniegelenk 1 zu verbinden.

Ersichtlich ist das Kniegelenk 1 in einer Vierachsgeometrie aufgebaut, wobei ein erstes Zwischenstück 5 und ein zweites Zwischenstück 6 umfasst sind. Das erste Zwischenstück 5 ist über eine im unteren Bereich des Unterschenkelverbindungsstücks 3 vorgesehene Achslagerung 7 mit dem Unterschenkelverbindungsstück 3 und über eine zweite Achslagerung 8 mit dem Oberschenkelverbindungsstück 2 verbunden und umfasst hier zwei parallele Verbindungsplatten. In der in Fig. 1 dargestellten maximalen Extension parallel hierzu ist das zweite Zwischenstück 6, das beispielsweise als Gabel ausgebildet sein kann, über eine dritte Achslagerung 9 mit dem Oberschenkelverbindungsstück 2 und eine vierte, im oberen Bereich des Unterschenkelverbindungsstücks 3 vorgesehene Achslagerung 10 mit dem Unterschenkelverbindungsstück 3 verbunden.

Eingeschaltet in das den grundsätzlichen Bewegungsablauf bei der Flexionsbewegung bzw. Extensionsbewegung definierende System von Teilstücken 2, 3, 5 und 6 ist zudem eine Schwungphasensteuerung 11. Diese umfasst einen Zylinder 12, in dem ein Kolben beweglich geführt ist, von dem hier nur die Kolbenstange 13 dargestellt ist. Die Funktionsweise von Schwungphasensteuerungen ist allgemein bekannt und muss hier nicht näher dargelegt werden.

Während die Kolbenstange 13 über eine Achslagerung 14 unmittelbar an das Oberschenkelverbindungsstück schwenkbar angelagert ist, ist der Zylinder 12 über eine Achslagerung 15 schwenkbar mit dem Zwischenstück 5 verbunden, wobei die Achslagerung 15 der ersten Achslagerung 7 benachbart ist. Insgesamt folgt dennoch, dass der dem Unterschenkelverbindungsstück 3 durch die nah an diesem vorgesehene Befestigung zugeordnete Zylinder 12 entweder über die Achslagerung 15 oder die Achslagerung 7 gegen das Unterschenkelverbindungsstück 3 schwenkbar gelagert ist.

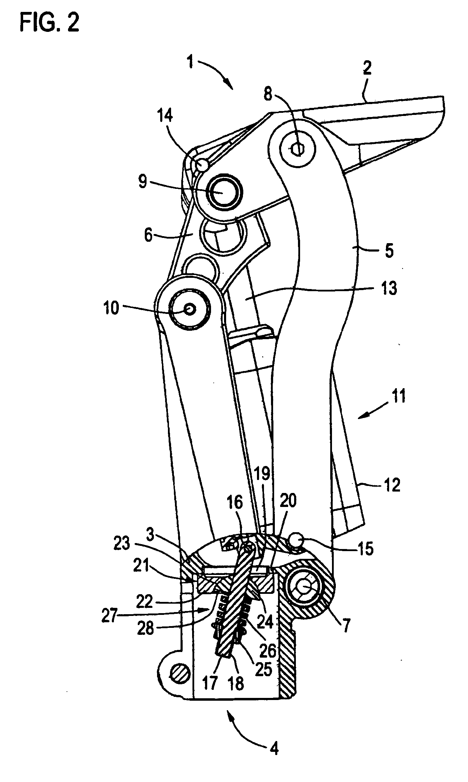

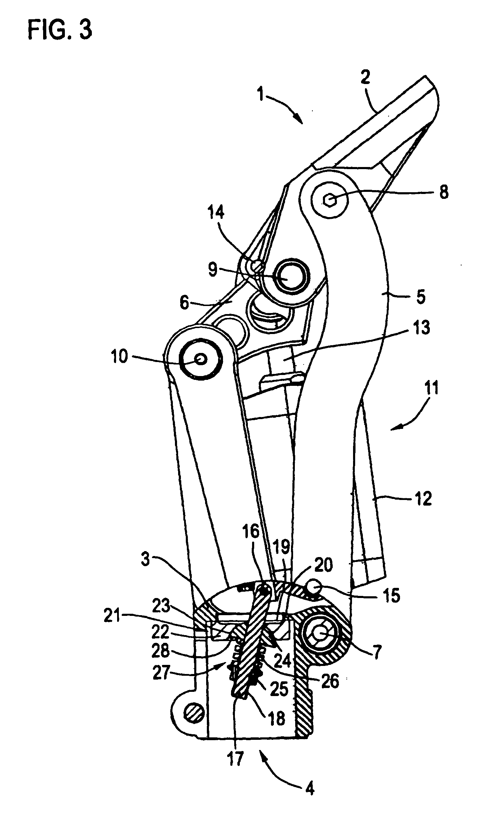

Ersichtlich zeigt der untere Teil der Fig. 1 (wie auch der der Figuren 2 und 3) eine geschnittene Ansicht. Dort ist ein Anlenkpunkt 16 am Zylinder 12 zu erkennen. An dem Anlenkpunkt 16 ist eine Gewindestange 17 mit einem Gewinde 18 gelenkig befestigt.

Dabei ragt die Gewindestange 17 in die Rohraufnahme 4 ein, was mittels einer Durchgangsöffnung 19 in einer oberen Fläche 20 des Unterschenkelverbindungsstücks 3 möglich ist. Auf der dem Zylinder 12 abgewandten Seite ist an den Rändern der Durchgangsöffnung gegengelagert eine Gelenkverbindung 21 vorgesehen, die vorliegend eine Gelenkpfanne 22 mit einer kalottenförmigen Mulde 23 umfasst, in die ein kalottenförmiger Gelenkkörper 24 eingesetzt ist. Der Gelenkkörper 24 besteht dabei aus Kunststoff und hat letztlich die Form einer Halbkugel. Ersichtlich weisen auch die Gelenkpfanne 22 und der Gelenkkörper 24 Durchgangsöffnungen auf, durch die die Gelenkstange 17 geführt ist. Während die Gewindestange 17 durch den Gelenkkörper 24 formschlüssig geführt ist, sind die Durchgangsöffnungen der Fläche 20 und der Gelenkpfanne 22 breiter gestaltet, um eine Verkippung der Gelenkstange 17 aufgrund einer relativen Verschwenkung von Zylinder 12 und Unterschenkelverbindungsstück 3 zu ermöglichen.

Auf das Gewinde 18 ist nun ferner eine Mutter 25 aufgeschraubt, die eine Abstützfläche für ein hier als Schraubenfeder 26 ausgebildetes Federmittel 27 darstellt. Auf der anderen Seite stützt sich die Feder 26 auf der ebenen, der Mulde 23 abgewandten Fläche 28 des Gelenkkörpers 24 und somit indirekt auf dem Unterschenkelverbindungsstück 3 ab. Über die Mutter 25 kann die Vorspannung der Feder 26 angepasst werden.

Wird der Zylinder 12 gegenüber dem Unterschenkelverbindungsstück 3 verschwenkt, so ändert sich der Abstand des Anlenkpunkts 16 von der benachbarten Fläche 20 des Unterschenkelverbindungsstücks 3. Im vorliegenden Fall wird nun bei einer Flexion, worauf mit Bezug auf Fig. 2 und Fig. 3 gleich noch näher eingegangen werden wird, der Zylinder 12 von der Fläche 20 entfernt, so dass die Gewindestange 17 mit der Mutter 25 durch die Durchgangsöffnungen nach oben gezogen wird und folglich die Feder 26 vorgespannt wird, so dass eine Vorspannkraft entsteht, die in Richtung der Extension wirkt. Es sei an dieser Stelle noch darauf hingewiesen, dass bei der dargestellten Ausführungsform die meisten Bestandteile dieser Vorrichtung zur Erzeugung einer Extensionskraft bei einer beginnenden Flexionsbewegung innerhalb der Rohraufnahme 4 angeordnet sind, mithin von außen nicht zu sehen sind und dort geschützt angeordnet sind.

Im Folgenden soll nun die Funktionsweise des Kniegelenks 1 mit Bezug auf die Figuren 2 und 3 näher erläutert werden.

Beim vorliegend dargestellten Ausführungsbeispiel tritt in einem ersten Flexionswinkelbereich, hier von 0° bis 8° eine starke Verschwenkung des Zylinders 12 um die durch die erste Achslagerung 7 definierte erste Achse auf. Dies wird durch Fig. 2 näher dargestellt, die das Kniegelenk 1 unter einem sehr kleinen Flexionswinkel zeigt. Trotzdem der Flexionswinkel noch sehr klein ist und der Kolben mit der Kolbenstange 13 kaum bewegt wurde, erkennt man, dass der Anlenkpunkt 16 bereits deutlich von der Fläche 20 des Unterschenkelverbindungsstücks 3 entfernt wurde. Dies bedeutet aber, dass die Schraubenfeder 26 stark gestaucht wird, mithin eine Vorspannkraft entlang der Längsrichtung der Gewindestange 17 entstanden ist. Diese Zugkraft bewirkt ein Drehmoment, das den Zylinder 12 (und das Zwischenstück 5) um die erste Achslagerung 7 nach hinten drückt. Hieraus resultiert wiederum eine Kraft, die die Achslagerung 14 der Kolbenstange 13 nach hinten drückt. Die Vorspannkraft wirkt der Flexionsbewegung entgegen, führt also dazu, dass das Gesamtsystem dazu tendiert, die maximale Extensionsstellung wieder einzunehmen. Die relative Verschwenkung zwischen dem Unterschenkelverbindungsstück 3 und dem Zylinder 12, also auch eine Verschwenkung der Gewindestange 17, wird durch die Gelenkverbindung 21 ausgeglichen, denn deren Konstruktion lässt in einem durch die Größe der Durchgangsöffnungen definierten Winkel ein freies Rotieren der Gewindestange 17 um den Mittelpunkt der Kalottengeometrie zu.

Wie bereits erwähnt, dreht sich der Zylinder 12 nur bis zum Ende des ersten Flexionswinkelbereichs, also bis ca. 8° Flexionswinkel (Kniegelenkbeugewinkel) erreicht sind, um die erste Achse der Achslagerung 7 und spannt somit die Schraubenfeder 26 weiter vor. Ab dem Flexionswinkel von ca. 8° vollführt das Oberschenkelverbindungsstück 2 eine rotatorische Bewegung, so dass der Abstand zwischen dem Anlenkpunkt 16 und der Fläche 20 im Wesentlichen gleich bleibt und sich die Vorspannkraft der Feder 26 kaum noch verändert - es kommt lediglich noch zu einer Drehbewegung zwischen der Gelenkpfanne 22 und dem Gelenkkörper 24.

Somit ist eine Erhöhung der Vorspannung und somit einer in Richtung der Extension wirkenden Kraft beim normalen Gehen nur in einem Bereich zwischen 0° und ca. 8° Flexionswinkel gegeben, so dass eine Beeinträchtigung der Schwungphasensteuerung 11 durch permanent ansteigende, in die Extensionsrichtung drückende Kräfte vermieden wird. Es ist somit ein Kräfteverlauf gegeben, der für eine bei kleinen Winkeln sehr schnell ansteigende Vorspannkraft sorge, die dann jedoch über einen zweiten Flexionswinkelbereich bei der weiteren für das Gehen relevanten Flexionsbewegung im Wesentlichen konstant bleibt.

Fig. 3 zeigt zur weiteren Illustration das Kniegelenk 1 nochmals unter einem größeren Flexionswinkel. Darin ist deutlich zu sehen, dass hauptsächlich eine Verschwenkung des Oberschenkelverbindungsstücks 2 erfolgt, wobei der Kolben immer tiefer im Zylinder 12 verschoben wird, wie an dem verkürzten freiliegenden Teil der Kolbenstange 13 ersichtlich ist. Die vorgespannte Feder 26 ändert ihre Vorspannung jedoch kaum noch. Dies steht im Gegensatz zu einer den Kolben im Zylinder 12 vorspannenden Feder, deren Vorspannung gerade diesen Bewegungsabschnitt ständig weiter zunehmen würde.

## Patentansprüche

1. Kniegelenk (1) für eine Prothese, umfassend eine Schwungphasensteuerung (11) mit einem mit einem Oberschenkelverbindungsstück (2) gekoppelten, gegen einen Zylinder (12) bewegbaren Kolben und dem mit einem Unterschenkelverbindungsstück (3) gekoppelten Zylinder (12), wobei der Zylinder (12) gegen das Unterschenkelverbindungsstück (3) um wenigstens eine Achse verschwenkbar ist, **dadurch gekennzeichnet, dass** der Zylinder (12) über einen achsfernen Anlenkpunkt (16) an ein sich an dem Unterschenkelverbindungsstück (3) abstützendes Federmittel (27) gekoppelt ist.

2. Kniegelenk nach Anspruch 1, **dadurch gekennzeichnet, dass** an dem Federmittel (27) eine Gelenkverbindung (11) zum Ausgleich einer relativen Bewegung zwischen dem Unterschenkelverbindungsstück (3) und dem Zylinder (12) vorgesehen ist.

3. Kniegelenk nach Anspruch 2, **dadurch gekennzeichnet, dass** die Gelenkverbindung (11) eine an dem Unterschenkelverbindungsstück (3) angeordnete, eine insbesondere kalottenförmige Mulde (23) umfassende Gelenkpfanne (22) und einen insbesondere kalottenförmigen, in die Mulde (23) einzusetzenden Gelenkkörper (24) umfasst.

4. Kniegelenk nach Anspruch 3, **dadurch gekennzeichnet, dass** die Gelenkpfanne (22) und der Gelenkkörper (24) eine Durchgangsöffnung für eine an dem Anlenkpunkt (16) mit dem Zylinder (12) gelenkig verbundene Stange (17) aufweist, um welche Stange (17) sich das als wenigstens eine Feder (26) ausgebildete, sich auf einer der Mulde (23) abgewandten Fläche (28) des Getenkkörpers(24) und einem an dem dem Zylinder (12) abgewandten Ende der Stange (17) vorgesehenen Anschlagmittel abstützende Federmittel (27) erstreckt.

5. Kniegelenk nach Anspruch 4, **dadurch gekennzeichnet, dass** die Stange (17) als Gewindestange (17) und das Anschlagmittel als auf dem Gewinde (18) verstellbare Schraubmutter (25) ausgebildet ist.

6. Kniegelenk nach einem der vorangehenden Ansprüche, **dadurch gekennzeichnet, dass** die Vorspannung des Federmittels (27) einstellbar ist.

7. Kniegelenk nach einem der vorangehenden Ansprüche, **dadurch gekennzeichnet, dass** bei einer Flexionsbewegung in einem vorgegebenen ersten, bei 0° beginnenden Flexionswinkelintervall, insbesondere einem bei einem Winkel kleiner als 20°, bevorzugt kleiner als 10°, endenden Flexionswinkelintervall, eine Verschwenkung des Zylinders (12) um die oder wenigstens eine Achse erfolgt und so eine ein gegenläufiges Drehmoment erzeugende Vorspannkraft in dem Federmittel (27) aufbaubar ist.

8. Kniegelenk nach Anspruch 7, **dadurch gekennzeichnet, dass** in einem an das erste Flexionwinkelintervall anschließenden zweiten, insbesondere bei 90° endenden Flexionswinkelintervall keine wesentliche Verschwenkung des Zylinders (12) mehr auftritt.

## Claims

1. A knee joint (1) for a prosthesis, comprising a swing-phase control (11) with a piston coupled to a thigh connecting element (2) capable of being moved against a cylinder (12) and the cylinder (12) coupled to a lower leg connecting element (3), wherein the cylinder (12) can be pivoted against the lower leg connecting element (3) about at least one axis,
**characterised in that**
the cylinder (12) is coupled via an articulation point (16) remote from the axis to a spring means (27) supported against the lower leg connecting element (3).

2. The knee joint according to claim 1,
**characterised in that**
a joint connection (11) is provided on the spring means (27) for the compensation of a relative movement between the lower leg connecting element (3) and the cylinder (12).

3. The knee joint according to claim 2,
**characterised in that**
the joint connection (11) comprises a joint socket (22) disposed on the lower leg connecting element (3) and providing especially a dome-shaped recess (23) and especially a dome-shaped joint body (24) for insertion into the recess (23).

4. The knee joint according to claim 3,
**characterised in that**
the joint socket (22) and the joint body (24) provide a through aperture for a rod (17) connected in an articulated manner at the articulation point (16) to the cylinder (12), around which rod (17) the spring means (27), formed as at least one spring (26), supported against a surface (28) of the joint body (24) facing away from the recess (23) and against a stopping means provided at the end of the rod (17) facing away from the cylinder (12), extends.

5. The knee joint according to claim 4,
**characterised in that**
the rod (17) is formed as a threaded rod (17) and the stopping means is formed as a threaded nut (25) adjustable along the thread (18).

6. The knee joint according to any one of the preceding claims,
**characterised in that**
the biasing of the spring means (27) is adjustable.

7. The knee joint according to any one of the preceding claims,
**characterised in that**,
in the case of a flexion movement within a specified, first flexion-angle interval beginning at 0°, especially a flexion-angle interval ending with an angle smaller than 20°, preferably smaller than 10°, a rotation of the cylinder (12) about the axis, or about at least one axis, takes place, and a biasing force can be built up in this manner in the spring means (27), generating a counter torque.

8. The knee joint according to claim 7,
**characterised in that**
a substantial rotation of the cylinder (12) no longer occurs in a second flexion-angle interval adjoining the first flexion-angle interval, especially ending at 90°.

## Revendications

1. Articulation de genou (1) pour une prothèse, comprenant une commande de phase d'inertie (11) avec un piston couplé avec une pièce de liaison de cuisse (2) et pouvant être déplacée vers un cylindre (12) et le cylindre (12) couplé avec une pièce de liaison de jambe (3), le cylindre (12) pouvant être basculé vers la pièce de liaison de jambe (3) autour d'au moins un axe, **caractérisée en ce que** le cylindre (12) est couplé au moyen d'un point d'articulation (16) éloigné de l'axe à un moyen de ressort (27) s'appuyant sur la pièce de liaison de jambe (3).

2. Articulation de genou selon la revendication 1, **caractérisée en ce qu'**une liaison articulée (11) est prévue sur le moyen de ressort (27) pour la compensation d'un déplacement relatif entre la pièce de liaison de jambe (3) et le cylindre (12).

3. Articulation de genou selon la revendication 2, **caractérisée en ce que** la liaison articulée (11) comprend une cavité glénoïde (22) disposée sur la pièce de liaison de jambe (3), comprenant une cavité (23) en particulier en forme de calotte et un corps d'articulation (24) en particulier en forme de calotte, à insérer dans la cavité (23).

4. Articulation de genou selon la revendication 3, **caractérisée en ce que** la cavité glénoïde (22) et le corps d'articulation (24) présentent une ouverture de passage pour une barre (17) reliée de façon articulée au cylindre (12) sur le point d'articulation (16), barre (17) autour de laquelle s'étend le moyen de ressort (27) conçu sous forme d'au moins un ressort (26), s'appuyant sur une surface (28), opposée à la cavité (23) du corps d'articulation (24) et un moyen de butée prévu sur l'extrémité, opposée au cylindre (12), de la barre (17).

5. Articulation de genou selon la revendication 4, **caractérisée en ce que** la barre (17) est conçue sous forme de tige filetée (17) et le moyen de butée sous forme d'écrou-vis (25) réglable sur le filetage (18).

6. Articulation de genou selon l'une des revendications précédentes, **caractérisée en ce que** la pré-tension du moyen de ressort (27) est réglable.

7. Articulation de genou selon l'une des revendications précédentes, **caractérisée en ce que**, lors d'un mouvement de flexion dans un premier intervalle d'ordre de flexion prédéfini, commençant à 0°, en particulier dans le cas d'un intervalle d'angle de flexion se terminant avec un angle inférieur à 20°, de préférence inférieur à 10°, on a un basculement du cylindre (12) autour de l'axe ou d'au moins un axe et ainsi une force de pré-tension générant un couple opposé peut être établie dans le moyen de ressort (27).

8. Articulation de genou selon la revendication 7, **caractérisée en ce qu'**il n'apparaît plus un basculement important du cylindre (12) dans un second intervalle d'angle de flexion se terminant en particulier à 90°, faisant suite au premier intervalle d'angle de flexion.
